# EUROPEAN PATENT SPECIFICATION

(11) **EP 1 515 615 B1**
(45) Date of publication and mention of the grant of the patent: **04.05.2011**
(21) Application number: 03740343.3
(22) Date of filing: 26.06.2003
(51) Int. Cl.: A61K 36/28

(54) **NUTRITIONAL COMPOSITION FOR DETOXIFICATION AND CANCER PREVENTION**
NAHRHAFTE ZUSAMMENSETZUNG ZUR ENTGIFTUNG UND VORBEUGUNG GEGEN KREBS
COMPOSITION NUTRITIONNELLE DESTINEE A LA DETOXICATION ET A LA PREVENTION DU CANCER

(30) Priority: 26.06.2002 US 180773
(43) Date of publication of application: 23.03.2005
(73) Proprietor: Nestec S.A., 1800 Vevey (CH)
(72) Inventor: MALNOE, Armand, CH-1066 Epalinges (CH); OFFORD CAVIN, Elizabeth, CH-1041 Poliez-Pittet (CH); CAVIN, Christophe, CH-1041 Poliez-Pittet (CH)
(74) Representative: Lock, Graham James
(86) International application number: PCT/EP2003/006720
(87) International publication number: WO 2004/002238

(56) References cited:
- EP-A- 0 600 544
- EP-A- 0 692 252
- EP-A- 0 850 569
- EP-A- 1 172 041
- WO-A-00/64282
- WO-A-01/03716
- WO-A-99/07239
- DE-A- 10 008 990
- FR-A- 2 789 269
- US-A- 4 770 880
- US-A- 5 725 895
- US-A- 6 156 355
- DATABASE WPI Section Ch, Week 198212 Derwent Publications Ltd., London, GB; Class D13, AN 1982-23456E XP002258781 & SU 827 007 A (CONSERVES IND PROD ASSCN), 1 October 1981 (1981-10-01)
- DATABASE WPI Section Ch, Week 199516 Derwent Publications Ltd., London, GB; Class B04, AN 1995-116001 XP002258782 & CN 1 079 880 A (SHEN Y), 29 December 1993 (1993-12-29)
- DATABASE WPI Section Ch, Week 199530 Derwent Publications Ltd., London, GB; Class B04, AN 1995-224780 XP002258783 & CN 1 087 237 A (CHUANYING BREWING CO), 1 June 1994 (1994-06-01)
- KELLOFF G J ET AL: "CHEMOPREVENTION OF PROSTATE CANCER: CONCEPTS AND STRATEGIES" EUROPEAN UROLOGY, S. KARGER AG., BASEL, CH, vol. 35, no. 5/6, 1999, pages 342-350, XP000872008 ISSN: 0302-2838
- HUBER WOLFGANG W ET AL: "Enhancement of the chemoprotective enzymes glucuronosyl transferase and glutathione transferase in specific organs of the rat by the coffee components kahweol and cafestol" ARCHIVES OF TOXICOLOGY, vol. 76, no. 4, May 2002 (2002-05), pages 209-217, XP002258779 ISSN: 0340-5761
- HUGGETT A C ET AL: "Chemoprotective effects of coffee and its components cafestol and Kahweol: Effects on xenobiotic metabolising enzymes" 16TH INTERNATIONAL SCIENTIFIC COLLOQUIUM ON COFFEE VOLS. 1 AND 2, 1995, pages 2) 65-72, XP001155739 16th International Scientific Colloquium on Coffee Vols. 1 and 2;Kyoto, Japan; April 9-14, 1995, Association Scientifique Internationale du Cafe (ASIC) {a}, 42, rue Scheffer, Paris, France ISBN: 2-900212-15-4 cited in the application

## Description

The present invention generally relates to nutritional compositions. More specifically, the present invention relates to nutritional compositions that include chicory extracts, to enhance health in humans and animals.

### Background of the invention

The need to enhance health in mammals has involved and continues to involve on-going research efforts and discoveries to prevent disease. In general, food, dietary or other nutritional sources are known to contain a number of constituents or agents which are believed to be capable of protecting against disease in humans and animals.

For example, oligosaccharides, such as inulin and various fructo-oligosaccharides, are reported to have prebiotic effects, such as promoting the growth of bifido- and lacto-bacteria in the gastrointestinal tract at the expense of pathogens including, for example, Clostridium perfringens. See, for example, Gibson et al., Food Microbiology, 11 (6), pp. 491-498 (1994). Although most reported experimentation has been carried out in vitro, there have been reports that these oligosaccharides have a similar effect in the gut of rats and humans. In this regard, it is generally known that the promotion of growth of bifido- and lacto-bacteria through the use of oligosaccharides can provide a variety of different beneficial effects on animals and humans, such as the prevention and/or treatment of diarrhea, increased growth, improved ability to breed or other like beneficial effects which enhance health.

Further, a primary mechanism by which dietary agents are believed to protect against cancer is by inducing enzymatic detoxification to prevent cancer causing agents from reacting or binding with critical target sites susceptible to cancerous cell growth. Research has indicated that bioactive compounds, such as cafestol and kahweol in coffee, can induce the total activity of glutathione-S-tranferase ("GST".) A.C. Huggett et al.; Chemoprotective Effects Of Coffee And Its Components Cafestol And Kahweol: Effects On Xenobiotic Metabolising Enzymes, Asic, 16 Colloque, Kyoto, pp. 65-72 (1995). The GST enzymes are generally believed to be an essential family of enzymes with tissue-specific distribution that are suitable for detoxification and thus cancer prevention. It is generally known that such detoxification enzymes can promote carcinogen inactivation by their ability to catalyse the reaction of a wide variety of electrophiles to glutathione. In this regard, it is generally believed that most activated carcinogens are electrophiles. Wim A. Nijhoff et al.; Carcinogenesis, vol. 16 no. 4, pp. 955-957 (1995).

Inulin or other dietary agents that are believed to promote health in humans and animals as discussed above, in general, can be derived from plants or other natural sources. For example, inulin is generally known to be purified from plants which contain high concentrations of inulin, such as chicory, Jerusalem artichoke, leek and asparagus. In this regard, the plant is typically purified or otherwise treated prior to use in order to enhance a plant's flavor, such as to eliminate, or at least minimize, a bitter flavor typically associated with chicory. See, for example, U.S 4,865,852. In chicory, the bitter flavor is believed to be derived from sesquiterpene lactones, such as lactucin and lactucopicrin. Further, it is believed that purification or other treatment of the plant prior to use can provide more accurate control of the amounts of bioactive agents, such as inulin, to be added to the diet of a human and/or animal.

In general, the purified plant product is prepared by hydrolysis with acids or enzymes. The hydrolysate is then collected and condensed to obtain the bioactive agent, such as inulin. For example, Japanese Patent Document No. 63-309147 discloses grinding chicory tubers, partially hydrolyzing them with acids, and then drying the hydrolysate with or without neutralization. However, the purification of, for example, fructo-oligosaccharides and inulin, can greatly add to the cost of the dietary product. Consequently, the use of such dietary products has been generally limited to specialty food or dietary products in humans and animals.

A need, therefore, exists for a nutritional composition that includes natural ingredients, such as chicory and/or extracts thereof, that are palatable to humans and animals, that can be inexpensively produced, and that can enhance health in humans and animals, such as the prevention of cancer.

### Summary of the invention

The present invention relates to nutritional compositions that can be utilized to enhance health in humans and animals, particularly the prevention of cancer. The nutritional compositions of the present invention at least include a phytochemical agent.

Applicants have discovered that the combination of fermentable dietary fiber, preferably prebiotic fibers, and phytochemical agents can be derived from a common source, such as a plant source including chicory. Using in vitro models of rodent hepatocytes, Applicants have demonstrated that chicory root extracts also contain phytochemicals (in addition to fermentable dietary fiber, preferably prebiotic fibers, such as inulin). The phytochemicals are capable of promoting GST enzyme activity, which can promote detoxification, stimulate an antioxidant defense or promote other like processes in a mammal which are believed to be responsible for the prevention of cancer. In this regard, Applicants have surprisingly found that the combination of prebiotic fibers (e.g., inulin), which have known anti-tumor properties, and chemopreventive phytochemicals can both originate from the same plant source that can be utilized to reduce the risk of cancer, particularly cancer associated with the gut or other tumor-sensitive sites.

To this end, in an embodiment of the present invention, a nutritional composition capable of reducing a risk of cancer is provided. The nutritional composition is defined in the appended claims. Preferably, the chicory extract in the nutritional composition includes an amount of at least 0.5 % by weight on a dry weight basis.

In an embodiment, the chicory extracts are as defined in the appended claims. In an embodiment, the chicory extracts, are from chicory pulp, chicory root, the like and combinations thereof.

In an embodiment, the phytochemical agent induces glutathione-S-transferase (GST) activity and is capable of promoting detoxification in tissue of the mammal. In another embodiment, the GST activity is capable of stimulating an antioxidant defense in tissue of the mammal.

The present invention also provides a pet food product as defined in the appended claims.

In still another embodiment of the present invention, a method of preparing a nutritional food product capable of reducing incidence of cancer in a mammal is provided. The process includes the steps as defined in the appended claims.

In an embodiment of the present invention, the chicory extract is processed by defatting the plant material to form a first plant extract and subsequently processing the first plant extract with ethyl acetate via acid hydrolysis to form the plant extract.

A method of reducing a risk of cancer in a mammal at risk of cancer is described herein

In addition, a method of increasing detoxification in tissue of a mammal is described herein.

It is believed that the increased enzymatic activity can promote detoxification thereby reducing a risk of incidence of cancer.

In addition, a method of stimulating an antioxidant defense in tissue of a mammal is described herein. It is believed that the increased enzymatic activity can stimulate the antioxidant defense thereby reducing a risk of incidence of cancer.

An advantage of the present invention is to provide an improved nutritional composition that can be utilized to reduce an incidence of cancer in mammals. In this regard, the nutritional composition is capable of promoting detoxification processes, antioxidant defenses, other like processes or combinations thereof in mammals which are believed to reduce an incidence of cancer.

Another advantage of the present invention is to provide an improved nutritional composition that includes a chicory extract as defined in the appended claims wherein the chicory extract contains a phytochemical(s) capable of inducing GST activity in order to reduce the risk of cancer in mammals at risk of cancer.

Yet another advantage of the present invention is to provide methods of producing improved nutritional compositions containing a plant material that can enhance the palatability of the nutritional composition while maintaining the enzymatic detoxification properties of the plant material.

A further advantage of the present invention is to provide methods of prevention against cancer in mammals that include the administration of an improved nutritional composition. The nutritional composition is defined in the appended claims.

Additional features and advantages of the present invention are described in, and will be apparent from, the following detailed description of the invention.

### Detailed description of the invention

The present invention relates to nutritional compositions as defined in the appended claims which can be utilized to effectively prevent cancer in humans and animals, particularly cancer associated with the gut, including, for example, colon cancer. Applicants have surprisingly discovered that certain plants and/or plant extracts thereof, such as chicory, contain phytochemicals which can stimulate detoxification pathways to prevent cancer, such as stimulate phase II enzymes including, glutathione-S-transferase.

In addition to phytochemicals as discussed above, plants, such as chicory, are known to contain fermentable dietary fibers, such as prebiotic fibers. The prebiotic fibers can include a number of suitable materials, , such as oligosaccharides including inulin. The fermentable dietary fibers are also believed to reduce cancer incidence, particularly in the colon. In this regard, Applicants believe that enhanced benefits with respect to cancer prevention in humans and animals can be realized due to the combined effect of fermentable dietary fibersand phytochemicals that can stimulate detoxification pathways.

Applicants have also demonstrated that the detoxifying properties of the nutritional composition of the present invention are essentially unaffected by the processing conditions under which the nutritional compositions are prepared pursuant to present invention. For example, purification of the plant material made pursuant to the present invention which can be utilized to reduce the bitterness of the plant extract and thus enhance human and animal palatability has negligible, if any, effect on the detoxification properties of the resultant purified product. In this regard, the desirable cancer prevention properties can result from essentially crude plant extracts, such as chicory. This can eliminate the need for expensive purification or other like treatment of a plant material to produce the desirable bioactive fraction(s).

As used herein, the term "bioactive agent" or other like terms, such as "bioactive fractions", means any constituent or constituents that can display biological activity, chemical activity or like activity in a mammal(s) that are capable of enhancing health in a mammal. Examples of bioactive agents include, for example, fermentable dietary fibers, such as prebiotic fibers, phytochemicals or the like.

As used herein, the term "prebiotic" or other like terms including "prebiotic fiber" means a substance or a constituent that can promote the growth of beneficial microorganisms in mammals.

As used herein, the term "phytochemical" or other like terms including "phytochemicals" and "phytochemical agent" means any chemical produced by a plant that is believed to impact health benefits to humans and/or animals, such as the prevention of cancer.

As used herein, the term "enzymatic activity" or other like terms, such as "enzyme activity", means any suitable enzyme which can act as a catalyst during any suitable biological, chemical or other like process which is believed to effect health in a mammal. For example, the increase of enzyme activity relating to glutathione-S-transferase can promote detoxification in tissue, stimulate an antioxidant defense in tissue or like processes which are believed to be responsible for the prevention of cancer.

The nutritional composition can include any suitable and compatible types and amounts of constituents such that the nutritional composition can be effectively utilized to prevent cancer. In an embodiment, the nutritional composition includes a chicory extract as defined in the appended claims when is capable of inducing or promoting GST enzyme activity, that is believed to be responsible for the detoxification of cancer causing agents. In addition to promoting detoxification via increased enzymatic activity, the phytochemical agent is also believed to stimulate antioxidant defenses, or stimulate other like processes in humans and/or mammals. As a result, the phytochemical agent is believed to be capable of enhancing health in the mammal, such as reducing the incidence of cancer.

In an embodiment, the nutritional composition comprises a chicory extract as defined in the appended claims. The extract can be made from any suitable part of the plant, for example, the root, the pulp, the like or combinations thereof. The extract is processed such that its flavor can be enhanced. For example, bitter flavors which are typically associated with plant materials, such as chicory, can be removed by processing the plant into an extract. The extract can also be prepared such that the amount of bioactive agent in the final extract product can be desirably controlled.

It should be appreciated that the chicory can be processed to form an extract in a variety of different and suitable ways. In general, the plant material, such as the chicory root, is ground, powdered or provided in any suitable form. The plant material can then be further processed in a number of different stages to produce the product extract. In an embodiment, a defatting procedure is performed on the plant material to produce an extract that results from fats removed from the plant material. The defatting procedure can be conducted under any suitable defatting process conditions with any suitable types and amounts of solvents including, for example, hexane.

In an embodiment, the resultant extract of the defatting procedure is further processed via acid hydrolysis to produce another type of plant extract that can be added to the nutritional composition of the present invention. The acid hydrolysis procedure is conducted under any suitable process condition with ethyl acetate.

The phytochemical agents can include any suitable type and amount such that it is capable of inducing GST activity that is believed to be responsible for the detoxification of carcinogens. In an embodiment, the phytochemical agent includes antioxidants, catechin, like constituents or combinations thereof. In an embodiment, the phytochemical agent of the chicory extract is capable of inducing phase II enzyme activity, such as GST enzyme activity. By inducing GST activity, the phytochemicals are believed to be capable of increasing detoxification in mammal tissues (e.g., increasing GST activity), capable of stimulating an antioxidant defense in mammal tissues (e.g., increasing levels of glutathione), or capable of enhancing health in mammals in other like mechanisms which are believed to result in, for example, the prevention of cancer.

It should be appreciated that the nutritional composition of the present invention can include a variety of different and suitable forms. In an embodiment, the nutritional composition can include a nutritional supplement, a food preparation for humans and/or animals, pet food or the like. The nutritional composition can be added to the food product in any suitable amount. In an embodiment, the food product includes the chicory extract of the nutritional composition in an amount of at least 0.5% by weight, preferably from about 0.5% to about 30% by weight, more preferably about 0.5% to about 2% by weight.

In an embodiment, the present invention provides a pet food product that includes a starch matrix and an effective amount of the chicory extract which includes a phytochemicals agent capable of inducing GST activity which is believed to enhance health in mammals, such as to prevent cancer as previously discussed. The pet food product of the present invention can include any suitable number, type and amount of constituents and be processed in any suitable way to form a desirable product form.

In an embodiment, the present invention includes a gelatinized cereal product which contains an amount of a chicory extract. The plant material at least includes a phytochemicals capable of stimulating GST activity in mammals which is believed to enhance health in the mammal as described above.

As described below, the remaining ingredients included in the gelatinized cereal product may be any suitable ingredients commonly used in gelatinized cereal products. In general, these ingredients include a starch source and a protein source. Suitable starch sources are, for example, grains such as corn, rice, wheat, beets, barley, oats, soy, and mixtures thereof. Suitable protein sources may be selected from any suitable animal or vegetable protein source. Examples include meat meal, bone meal, fish meal, soy protein concentrates, milk proteins, gluten, and the like. The choice of the starch and protein sources will be largely determined by the nutritional needs of the animal or human, palatability considerations, the type of cereal product produced or other like considerations. Various other ingredients, for example, sugar, salt, spices, seasonings, vitamins, minerals, flavoring agents, fats and the like may also be incorporated into the gelatinized cereal product as desired.

The gelatinized cereal product may be produced in many different ways as desired. However, for a dried cereal product, an especially suitable way of producing the product is extrusion cooking. This may be done as is well known in the art. For example, in one suitable process, a feed mixture is fed into a preconditioner. The feed mixture is primarily made up of a starch source, a protein source, and the plant material, such as, chicory. In an embodiment, the chicory includes at least about 1% by weight of the feed material, preferably at least about 2% by weight. In an embodiment, the plant material ranges from about 10% to about 20% by weight, preferably about 10% by weight. In the preconditioner, water or steam, or both, is mixed into the feed mixture. A sufficient amount of water or steam is mixed into the feed mixture to moisten the feed mixture. If desired, the temperature of the feed mixture may be raised in the preconditioner to about 60°C to about 90°C by weight. A suitable preconditioner is described in U.S 4,752,139. It should be appreciated that the use a preconditioner is not required.

The moistened feed leaving the preconditioner is then fed into an extruder. The extruder may be any suitable single or twin screw and cooking extruder. Suitable extruders may be obtained from Wenger Manufacturing Inc., Clextral SA, Bühler AG, and the like. During passage through the extruder, the moistened feed passes through a cooking zone, in which it is subjected to mechanical shear and is heated. In an embodiment, the moistened feed is heated up to a maximum temperature of up to about 150°C, and a forming zone. The gauge pressure in the forming zone is about 300 kPa to about 10 MPa as desired. If desired, water or steam, or both, may be introduced into the cooking zone. During passage through the extruder, the starch source of the moistened feed is gelatinized to provide a gelatinized matrix structure primarily of starch, protein and the plant material, such as chicory. The gelatinized matrix leaving the extruder is forced through a suitable die for example, a die as described in European Patent Application No. 0665051, the disclosure of which is herein incorporated by reference. A shaped extrudate, which has a cross-sectional shape corresponding to that of the orifice of the die, leaves the die. Depending upon the conditions in the extruder and the starch source used, the shaped extrudate expands to a greater or lesser extent. The shaped extrudate is then cut into pieces using blades. The individual pieces are then dried and, if desired, coated with protective or flavoring agents, or both. After cooling, the pieces may be packed into suitable packages. Alternatively, the individual pieces may be formed into flakes and then dried.

Depending upon the ingredients used, the gelatinized cereal product may be in the form of dried kibbles suitable for use as pet foods, expanded pieces suitable for use in breakfast cereals, flakes suitable for use in breakfast cereals, and the like.

It is also possible to produce a dried cereal product by mixing together water and the ingredients of cereal product, for example, by mixing in a preconditioner. The wet mixture may then be shaped into a desired shape by using, e.g. shaping rollers. The shaped mixture may then be baked in an oven, at any suitable temperature. In an embodiment, the temperature ranges from about 220°C to about 280°C for a suitable baking time. In an embodiment, the baking time ranges from about 10 minutes to about 1 hour. The dried cereal product has the appearance of a baked biscuit.

If it is desired to produce a simulated meat product which may be used in canned pet foods, any suitable process can be used. For example, the processes can include those described in U.S 4,781,939 and 5,132,137. In these processes, a protein source, especially a meat material, is emulsified. The meat material may be any suitable source of animal protein including for example, the muscular or skeletal meat of mammals, poultry, and fish or meat by-products, such as hearts, liver, kidneys, tongue and the like, or meat meals. Vegetable protein sources may also be included if desired. The exact composition may be selected according to cost and the desired flavor. The emulsification may be carried out in any suitable equipment.

The dried chicory is added to the emulsion. Also, if desired or needed, additional protein may be added to the emulsion. The additional protein may be any protein source as mentioned above. The exact choice will depend upon availability, cost and palatability. The additional protein can be added in any suitable amount. In an embodiment, the additional protein can be added in an amount ranging from about 5% to about 35% by weight.

If desired or required, fats may also be added to the emulsion. Usually the amount of fat in the emulsion must be controlled to facilitate processing and to obtain an acceptable product. However, the meat material may well contain the desired amount of fats and hence adjustment may not be necessary. Typically, at this stage the emulsion contains a maximum fat level of about 25% by weight. In an embodiment, the amount of fat in the emulsion is in the range of about 5% to 15% by weight, more preferably about 7% to about 12% by weight. The mass ratio of protein to fat in the emulsion is preferably about 1:1 to about 7:1. If added, the fats may be any suitable animal fats, such as tallow, or may be vegetable fats.

Additional ingredients such as sugars, salts, spices, seasonings, flavoring agents, minerals, and the like may also be added to the emulsion. In an embodiment, the amount of additional ingredients used ranges from about 1% to about 5 % by weight of the gelatinized cereal product. Water may also be added to provide from about 45% to 80% by weight moisture in the emulsion. If sufficient moisture is present in the meat material, water need not be added.

Once mixed, the emulsion is preferably fed through a vacuum stuffer, or similar de-aeration apparatus, to de-aerate the emulsion. This removes air which may otherwise cause disruption of the formulated emulsion product and reduce its meat-like appearance. The emulsion is then fed to an emulsion mill which subjects the emulsion to rapid mechanical heating and shearing. Any suitable emulsion mill may be used including, for example, the emulsion mill disclosed in U.S 5,132,137. Other suitable emulsion mills are commercially available under the trade name of TRIGONAL and may be obtained from Siefer Machinenfabrik GmbH & Co KG. Bahnhofstrasse 114, Postfach 101008., Velbert 1, Germany.

The temperature of the emulsion can be raised to the desired coagulation temperature in the emulsion mill in a few seconds. In an embodiment, the coagulation temperature ranges from about 100°C to about 120°C. In an embodiment, the temperature ranges from about 45°C to about 75°C as described in U.S 5,132,137. In general, the mechanical energy generated in the emulsion mill will be sufficient to heat the emulsion to the desired temperature but this may be supplemented by the injection of superheated steam.

The heated emulsion leaving the emulsion mill can be transferred to a holding tube. In the holding tube, the heated emulsion coagulates while moving slowly along the holding tube. The residence time of the heated emulsion in the holding tube is sufficient for the emulsion to have coagulated into a firm emulsion product upon reaching the exit of the holding tube.

The firm emulsion product leaving the holding tube is then transferred to a cutter where it is cut into pieces, such as chunks, of size suitable for use in a pet food. The chunks have the appearance and texture of meat. The chunks may be subjected to flaking if desired. The chunks may also be formulated into a chunk-in-gravy type of product.

Other procedures for producing chunks are known and may be used, such as extruding a feed mixture, cooking the feed mixture in a steam oven, and the cutting of the cooked extrudate into chunks.

If it is desired to produce a canned pet food in the form of a meat loaf, a meat batter may be prepared by emulsifying a suitable meat material to produce a meat emulsion. The meat material may be any suitable meat source, for example, as described above. Suitable gelling agents including gums, such as kappacarrageenan, locust bean gum, guar gum, xanthan gum or the like, may be added to the meat emulsion. In an embodiment, no more than about 2% by weight of gum is used. The dried plant material, such as chicory is then added to the meat emulsion.

Additional ingredients such as sugars, salts, spices, seasonings, flavoring agents, minerals, and the like may also be added to the meat emulsion. The amount of additional ingredients used is preferably such that they make up about 0.25% to about 5% by weight of the meat batter. Water may also be added the meat emulsion to provide from about 70% to about 85% by weight. If sufficient moisture is present in the meat material, water need not be added.

The meat emulsion is then heated to a temperature above about 65°C in a mixer-cooker. Steam may be injected into the meat batter if desired. The heated meat emulsion is then again emulsified to provide a loaf batter and the loaf batter maintained at a temperature above about 60°C until filling into cans.
It should be appreciated that the gelatinized cereal product may be produced by any suitable process and not only those described above. Other types of oligosaccharides may also be included in the gelatinized cereal product, such as fructo oligosaccharide and soy oligosaccharide. The soy oligosaccharides may be added in the form of soy meal or other suitable soy source.
The cereal products may be in any suitable form including; for example, dried, semi-wet and wet However, the matrix that makes up the cereal product must be gelatinized in order to remove or destroy the sesquiterpene compounds that may be present in the plant material. It should be appreciated that the cereal product of the present invention can be made for human and/or animal consumption.
Examples of pet food products made pursuant to an embodiment of the present invention are illustrated below.

### Example 1

A feed mixture is made up of about 58% by weight of corn, about 6% by weight of corn gluten, about 23% by weight of meat and meal, dried chicory and salts, vitamins and minerals making up the remainder. The dried chicory is in the form of a chicory extract made pursuant to an embodiment of the present invention and added in an amount of about 5% or less.
The feed mixture is fed into a preconditioner and moistened. The moistened feed is then fed into an extruder-cooker and gelatinized. The gelatinized matrix as it leaves the extruder is forced through a die and extruded, thus forming an extrudate. The extrudate is cut into pieces suitable for feeding to dogs, dried at about 110°C for about 20 minutes, and cooled to form pellets. It should be appreciated that part or a totality of the fat mix, or of the fat and oils used, can be added at a later stage, for example, as a coating.
The added chicory can enhance health in a mammal, such as preventing cancer by, for example, promoting and/or stimulating detoxification, antioxidant defenses or the like in tissue as previously discussed.

### Example 2

A dry pet food is prepared like the dried pet food of Example 1. It further includes an additional ingredient typically associated with enhancing the palatability of the dry pet food suited to cats. The added chicory can enhance health in a mammal, such as preventing cancer by, for example, stimulating and/or promoting detoxification, antioxidant defenses or the like in tissue, as previously discussed.

### Example 3

A feed mixture includes about 58% by weight of corn, about 6% by weight of corn gluten, about 23% by weight of chicken meal, dried chicory, salts, vitamins and minerals that make up the remainder. The chicory is added in an amount of about 5% or less. As previously discussed, the added chicory can stimulate enzymatic activity which is believed to enhance health in the animal, such as preventing cancer by, for example, promoting and/or stimulating detoxification, antioxidant defenses or the like in tissue.

The feed mixture is fed into a preconditioner and moistened. The moistened feed is then fed into an extruder-cooker and gelatinized. The gelatinized matrix as it leaves the extruder is forced through a die and extruded, thus forming an extrudate. The extrudate is cut into pieces suitable for feeding to cats, dried at about 110°C for about 20 minutes, and cooled to form pellets. At this stage, a lyophilized powder of one or more strains of the *Lactobacillus* species, such as *Lactobacillus rhamnosus* NCC2583 (CNCM I-2449), *Lactobacillus acidophilus* NCC2628 (CNCM I-2453) and *Enterococcus faecium* SF68 (NCIMB 10415), is applied to the pellets. A sufficient amount of the powder is applied to the pellets such that the corresponding dietary intake amount for the cat is from about 1.0E+07 to about 1.0E+9 cfu / day. In this regard, a portion of the powder is mixed into a first mass of pellets which are subsequently bagged. A second portion of the powder is measured and mixed with a lipid carrier which is then sprayed on to a second mass of pellets. The pellets are bagged after the coating has dried sufficiently at 50-60C for some minutes.

### Example 4: Canned Pet Food and Supplement

A mixture is prepared from about 73% of poultry carcass, pig lungs and beef liver (ground), about 16% of wheat flour, about 2% of dyes, vitamins, and inorganic salts. This mixture is emulsified at 12°C and extruded into the form of a pudding. Dried chicory in the form of an extract made pursuant to an embodiment of the present invention is added to the emulsion in an amount of about 5% or less. As previously discussed, the added chicory can enhance health in a mammal. The emulsion is then cooked at a temperature of 90°C. It is cooled to 30°C and then cut into chunks. About 45% of the chunks are mixed with about 55% of a sauce that is prepared from about 98% of water, about 1% of dye and about 1% of guar gum. Tinplate cans are filled with the chunk and sauce mixture and sterilized at 125°C for about 40 minutes.

As a probiotic supplement to be mixed with the pet-food before serving, additional packaging in sachet form with strains of the following *Lactobacillus* species are provided *Lactobacillus rhamnosus* NCC2583 (CNCM I-2449), *Lactobacillus acidophilus* NCC2628 (CNCM I-2453) or *Enterococcus faecium* SF68 (NCIMB 10415). The corresponding dietary intake of the supplement for the pet is from about 106-10¹² cfu/day, depending on the type of pet, e.g., a cat or a dog, and on the pet's physical factors, such as body mass. The supplement is packaged such that it is removably attached to the can, together with feeding directions.

Experimental testing as described in detail below was conducted to demonstrate the effectiveness of the present invention.

### In Vitro Testing

The inventors have conducted a number of experimental tests to demonstrate the beneficial effects of the present invention. In general, rat primary hepatocyte cultures were prepared and treated with varying amounts of chicory extracts over a period of about 48 hours. Experiments were then conducted to determine cytotoxic effects and effects of inducing or stimulating enzymatic activity in mammals which is believed to be responsible for enhancing the health of the mammal, such as by preventing and/or treating cancer by, e.g, promoting detoxification, antioxidant defenses or the like in tissue as previously discussed. The preparation and experimental testing procedures and results are discussed below in greater detail.

### Preparation of Cell Culture

Primary isolated hepatocytes were obtained by perfusion of the liver of Sprague-Dawley rats (250g) with a collagenase solution as described in, for example, Sidhu J.S. et al.; Archive of Biochemistry & Biophysic; 301, pp.1-11, 1993. Cell viability, estimated by Trypan Blue exclusion test, was found to range between about 90 to about 95%.

The cells were seeded at a density of 10⁶ cells/ml on 60mm plastic tissue culture dishes in 3ml of William's medium supplemented with 2mM L-glutamine, 10mM Hepes pH 7.4, ITS+, 15000U Penicillin/Streptomycin, 100nM Dexamethasone and 5% Fetal bovine serum (Hi-clone). Hepatocytes were allowed to attach for two hours and then washed with EBSS to remove debris and unattached cells. Fresh serum-free medium containing 25 nM of dexamethasone was added and an overlay of matrigel (233 µg/ml) was then applied. Fresh matrigel was added to the cultures every two days following medium change. To study the effects of chicory extracts made pursuant to an embodiment of the present invention, the components were added to culture media 24 hours after cell seeding over a period of 48 hours.

### Preparation of Chicory Extracts

Four different chicory extracts, namely Extracts A-D, were prepared pursuant to an embodiment of the present invention. Initially, a 40 gram (g) and a 10g sample of chicory ground to powder form were each sieved at 0.5mm. The samples were then processed to remove fats by mixing the samples with hexane for about thirty minutes at room temperature. 600 milliliters (ml) of hexane was added to the 40g chicory sieved sample, and 150ml were added to the 10g-sieved sample. The hexane was evaporated under vacuum at about 50°C to form Extract A.

Extract B was prepared by first defatting 40g of ground chicory powder as previously discussed. The defatted sample was hydrolyzed in 300ml of an acid, such as HCl, in a boiling water bath for about 20 minutes. After cooling and centrifugation (8000 rpm, 5 minutes, 10°C), the solution was extracted with 150ml of a solvent, such as ethyl acetate, which is commercially available, such as from MERCK. The solvent is evaporated to dryness. After further drying on anhydrous sodium sulfate under vacuum at about 50°C, Extract B was formed.

Extracts C and D were prepared as follows. First, a 10g sample of ground chicory powder was defatted as previously discussed. The second part of the defatted powder is extracted with about 250ml of a solvent/water mix, such as a 1:1 volume ratio of MeOH and water in solution. The extraction is performed under stirring at room temperature (e.g., about 20°C to about 25°C) for about 30 minutes. After centrifugation, the solution is divided into two equal volumes. To the first volume part of the solution, the organic solvent is evaporated under vacuum at about 50°C. The remaining aqueous phase is freeze-dried to form Extract C.

The second volume part of the solution is treated with about 2g of polyvinylpolypyrrolidone under stirring for about 30 minutes to trap polyphenols. The adsorbant material is removed by filtration, centrifugation or the like. The organic solvent is evaporated under vacuum at about 50°C. The remaining aqueous phase is freeze-dried to form Extract D.

### Cytotoxic Effects of Chicory Extract

Experimental tests were conducted to determine non-cytotoxic doses of the chicory extract using MTT assay. Rat hepatocytes were treated with varying amounts of chicory extract B, the preparation of which was previously discussed. The test results indicated that cytotoxicity of chicory extracts was observed at about 200 micrograms/milliliter or more.

### Western Blot Analysis

Whole protein extracts from cell cultures treated for 48 hours with chicory extracts (A-D) were resolved by SDS-PAGE on 10 % gels (10 or 25 µg protein /lane for glutathione-S tranferase ("GST") and heat shock protein ("HSP") analyses respectively) using the discontinuous buffer system of Laemmli and transferred electrophoretically to nitrocellulose membranes. Blots were incubated for 1 hour in a solution of 5% dried milk in PBS containing 0.1 % Tween to block protein binding sites. The blots were incubated with rabbit polyclonal antibodies raised against rat GST Ya, Yc, Yb1, Yb2, Yp (BIOTRIN) and rat GST Yc2 (made available from Dr. J. Hayes of Dundee University). The antibody against rat GST Yc2 is a polyclonal antibody known to cross-react with other rat GST alpha subunits. See, Hayes J.D. et al., Biochemical :J, 279, 385-398, 1991; and Cavin C. et al., Carcinogenesis 19, 1369-1375, 1998.

On Western blots, two bands are typically visible, with the upper and lower bands corresponding respectively to Yc1 and Yc2 subunits. The blots were also incubated with the rat polyclonal antibody raised against Heme-oxygenase-1 (HO-1) also called HSP 32 (stressgene, AMS). Blots were subsequently probed with a secondary antibody conjugated to horseradish peroxidase. The complex was visualized indirectly by chemiluminescence using an ECL western blotting detection kit (made available from AMERSHAM LIFE SCIENCE, England). Protein concentrations were measured using the method of Bradford which was standardized using BSA.

The test results indicated that Chicory Extract B which was made with ethyl acetate as previously discussed, increased the expression of some GSTs subunits (Yc2, Yb1 Yp) in rat hepatocytes as compared to controls and cells treated with Chicory Extracts A, C and D.

### Enzymatic Assays

GST activities of cytosolic fractions were assayed as described in Habig W.G. et al, Journal of Biological Chemistry 249, 7130-7139 (1974). 1-chloro-2,4-dinitrobenzene (CDNB) was used as a substrate to measure general GST activity in rat primary cultures treated with varying amounts of chicory extracts, namely Extracts A-D as previously discussed. The incubations were performed at 30°C. Further, ethacrynic acid was used as a substrate to measure specific GST-P enzymatic activity in rat primary cultures treated with varying amounts of chicory extracts, namely Extracts A-D as previously discussed.

The test results indicated that Extract B resulted in an increased level of GST activity in rat hepatocytes as compared to controls and Extracts A, C and D.

### Determination of Total Glutathione

Total glutathione level in cells was measured by enzymatic recycling as described in Gallagher E.P. et al., In Methods in toxicology Vol. IB, 349-366 (1994). Glutathione (GSH) is measured with a kinetic assay which utilizes the continuous glutathione reductase-catalyzed reduction of the sulfhydryl reagent 5,5'-dithiobis-2-nitrobenzoic acid (DTNB; Ellman's reagent) to the chromophoric product 2-nitro-5-thiobenzoic acid. Detection of the chromophore is monitored spectrophotometrically at 412nm.

Rat primary hepatocytes were treated for 48 hours with varying concentrations (50, 100 and 200 µg/ml) of chicory Extract A (hexane), Chicory Extract B (ethyl acetate) and chicory Extract C (MetOH/H20). Cells were washed and resuspended in 125 microliters of 20mM 5-SSA. Cellular GSH was then released by sonification and samples centrifuged at 10000g for two minutes at room temperature. The supernatant containing free GSH was then used for the determination of total glutathione. Sample or GSH standard were mixed with 700µl of 125 mM sodium phosphate containing 6.3 mM EDTA (pH 7.5), 100µl of 6 mM DTNB and 20µl of 20mM NADPH. After equilibration of cuvettes to 25°C, 10µl of GSSG reductase (50 U/ml) is added to measure the formation of 2-nitro-5-thiobenzoic acid at 412 nm. A sample blank lacking GSH is run separately and the resulting background formation of product formation is subtracted from the sample value prior to GSH quantification.

The test results indicated that chicory extract B resulted in increased cellular GSH levels in rat hepatocytes as compared to controls and chicory extracts A and C. Further, the test results indicated that the cellular levels of GSH increased with increased amounts of chicory extract B.

### Effect of Food Processing

Test samples were prepared to evaluate the effects food processing on the detoxification properties of the plant material constituent of the resultant food product. The test was conducted on a pet food that contained about 30% by weight of chicory made in accordance with an embodiment of the present invention. The pet food was then processed by extraction with ethyl acetate in accordance with an embodiment of the present invention. The resultant extract was added to rat hepatocyte cultures in varying amounts, namely 100 µg/ml, 200 µg/ml and 400 µg/ml. Western blot analysis was then conducted to determine the effects of the chicory extract on the GST activity of the hepatocyte cultures.

The test results indicated that the food processing procedure did not reduce and in fact enhanced the detoxification properties of the plant material, namely chicory. In this regard, an increased level of GST activity was measured with respect to the varying amounts of chicory extract added to the cultures. Further, the level of GST activity increased with increasing amounts of chicory extract.

## Claims

1. A biologically effective amount of a chicory extract for use in reducing a risk of cancer in a mammal, wherein
(i) the chicory extract is extracted from chicory by acid hydrolysis and solvent extraction with ethyl acetate,
(ii) the chicory extract includes a phytochemical agent that induces glutathione-S-transferase activity in the mammal.

2. The chicory extract for use of claim 1 wherein the chicory extract is extracted from chicory root, chicory pulp and combinations thereof.

3. The chicory extract for use of claim 1 wherein the phytochemical agent is selected from the group consisting of an antioxidant, catechins and combinations thereof.

4. The chicory extract for use of claim 1 wherein the glutathione-S-transferase activity is capable of promoting detoxification in tissue of the mammal.

5. The chicory extract for use of claim 1 wherein the glutathione-S-transferase activity is capable of stimulating an antioxidant defense in tissue of the mammal.

6. The chicory extract for use of claim 1 wherein the chicory extract is formulated in a nutritional composition selected from the group consisting of a nutritional supplement, a nutritionally complete food product, a food preparation, a cereal product and a pet food.

7. The chicory extract for use of claim 6, wherein the pet food comprises a starch matrix.

8. A pet food product for use in reducing a risk of cancer in a mammal comprising a chicory extract extracted from chicory by acide hydrolysis and solvent extraction with ethyl acetate wherein the chicory extract includes a phytochemical agent that induces glutathione-S-transferase activity in the mammal

9. The pet food product for use of claim 8 wherein the product comprises an amount of at least 0.5% of the chicory extract on a dry weight basis.

10. The pet food product for use of claim 8 wherein the phytochemical agent is selected from the group consisting of an antioxidant, catechins and combinations thereof.

11. A method of preparing a nutritional food product capable of reducing a risk of cancer in a mammal, the process comprising the steps of:
processing chicory by acid hydrolysis and solvent extraction with ethyl acetate to form a chicory extract including a phytochemical agent capable of inducing glutathione-S-transferase activity in the mammal; and
processing the extract and one or more food ingredients to form the nutritional food product that includes at least 0.5% by weight of the extract.

12. The method of claim 11 wherein the chicory extract is processed by defatting chicory to form a first plant extract and subsequently processing the first plant extract with ethyl acetate via acid hydrolysis to form the plant extract.

13. A biologically effective amount of a chicory extract extracted from chicory by acid hydrolysis and solvent extraction with ethyl acetate that contains a phytochemical agent for use in increasing detoxification in tissue of a mammal, wherein the phytochemical agent induces glutathione-S-transferase activity in the mammal.

14. The chicory extract for use of claim 13 wherein the nutritional composition comprises about 0.5% to about 2% by weight of the extract.

15. A biologically effective amount of a chicory extract that contains a a phytochemical agent for use in stimulating an antioxidant defence in tissue of a mammal wherein the phytochemical agent induces an glutathione-S-transferase activity in the mammal, and wherein the chicory extract is extracted from chicory by acid hydrolysis and solvent extraction with ethyl acetate.

16. The chicory extract for use of claim 15 wherein the nutritional composition comprises about 0.5% to about 2% by weight of the extract.

17. The chicory extract for use of claim 15 wherein the increased glutathione-S-transferase activity results in an increased level of glutathione.

## Patentansprüche

1. Eine biologisch wirksame Menge eines Chicoréeextrakts für den Gebrauch zur Reduzierung eines Krebsrisikos bei einem Säugetier, wobei
(i) der Chicoréeextrakt durch Säurehydrolyse und Lösemittel-Extraktion mit Ethylacetat von Chicorée extrahiert wird,
(ii) der Chicoréeextrakt ein phytochemisches Agens einschließt, das Glutathion-S-Transferase-Aktivität in dem Säugetier induziert.

2. Der Chicoréeextrakt für den Gebrauch nach Anspruch 1, wobei der Chicoréeextrakt extrahiert ist aus der Chicoréewurzel, dem Chicoréemark und Kombinationen daraus.

3. Der Chicoréeextrakt für den Gebrauch nach Anspruch 1, wobei das phytochemische Agens aus der Gruppe bestehend aus einem Antioxidans, Catechinen und Kombinationen daraus ausgewählt ist.

4. Der Chicoréeextrakt für den Gebrauch nach Anspruch 1, wobei die Glutathion-S-Transferase-Aktivität geeignet ist, die Entgiftung im Gewebe des Säugetiers zu begünstigen.

5. Der Chicoréeextrakt für den Gebrauch nach Anspruch 1, wobei die Glutathion-S-Transferase-Aktivität geeignet ist, eine Antioxidans-Abwehr im Gewebe des Säugetiers zu stimulieren.

6. Der Chicoréeextrakt für den Gebrauch nach Anspruch 1, wobei der Chicoréeextrakt gestaltet ist in einer nahrhaften Zusammensetzung, ausgewählt aus einer Gruppe bestehend aus einem Nahrungsergänzungsmittel, einem vollwertigen Nahrungsmittelprodukt, einer Nahrungszubereitung, einem Getreideerzeugnis und einem Tiernahrungsmittel.

7. Der Chicoréeextrakt für den Gebrauch nach Anspruch 6, wobei das Tiernahrungsmittel eine Stärkematrix umfasst.

8. Ein Tiernahrungsmittelprodukt für den Gebrauch zur Reduzierung eines Krebsrisikos bei einem Säugetier umfasst ein Chicoréeextrakt, extrahiert von Chicorée durch Säurehydrolyse und Lösemittel-Extraktion mit Ethylacetat, wobei der Chicoréeextrakt ein phytochemisches Agens einschließt, das Glutathion-S-Transferase-Aktivität in dem Säugetier induziert.

9. Ein Tiernahrungsmittelprodukt für den Gebrauch nach Anspruch 8, wobei das Produkt eine Menge von wenigstens 0,5 % des Chicoréeextrakts auf Trockengewichtsbasis umfasst.

10. Ein Tiernahrungsmittelprodukt für den Gebrauch nach Anspruch 8, wobei das phytochemische Agens aus der Gruppe bestehend aus einem Antioxidans, Catechinen und Kombinationen daraus ausgewählt ist.

11. Eine Methode zur Zubereitung eines nahrhaften Nahrungsmittelproduktes, das geeignet ist, ein Krebsrisiko bei einem Säugetier zu reduzieren, wobei das Verfahren die Schritte umfasst:
Verarbeitung von Chicorée mittels Säurehydrolyse und Lösemittel-Extraktion mit Ethylacetat, um ein Chicoréeextrakt zu schaffen, das ein phytochemisches Agens einschließt, das geeignet ist, Glutathion-S-Transferase-Aktivität bei einem Säugetier zu induzieren; und
Verarbeitung des Extrakts und eines oder mehr Nahrungsmittelbestandteilen, um das nahrhafte Nahrungsmittelprodukt zu schaffen, das wenigstens 0,5 % des Extraktgewichts einschließt.

12. Die Methode nach Anspruch 11, wobei der Chicoréeextrakt durch Entfettung des Chicorée verarbeitet wird, um einen ersten Pflanzenextrakt zu schaffen und anschließend den ersten Pflanzenextrakt mit Ethylacetat zu verarbeiten mittels Säurehydrolyse, um den Pflanzenextrakt zu schaffen.

13. Eine biologisch wirksame Menge eines Chicoréeextrakts, extrahiert von Chicorée durch Säurehydrolyse und Lösemittel-Extraktion mit Ethylacetat, der ein phytochemisches Agens enthält zum Gebrauch bei der Beschleunigung der Entgiftung im Gewebe eines Säugetiers, wobei das phytochemische Agens bei dem Säugetier Glutathion-S-Transferase-Aktivität induziert.

14. Der Chicoréeextrakt für den Gebrauch nach Anspruch 13, wobei die nahrhafte Zusammensetzung etwa 0,5 % bis etwa 2 % des Extraktgewichts umfasst.

15. Eine biologisch wirksame Menge eines Chicoréeextrakts, das ein phytochemisches Agens für den Gebrauch zur Stimulation einer Antioxidansabwehr im Gewebe eines Säugetiers beinhaltet, wobei das phytochemische Agens eine Glutathion-S-Transferase-Aktivität bei dem Säugetier induziert, und wobei der Chicoréeextrakt durch Säurehydrolyse und Lösemittel-Extraktion mit Ethylacetat von Chicorée extrahiert ist.

16. Der Chicoréeextrakt für den Gebrauch nach Anspruch 15, wobei die nahrhafte Zusammensetzung etwa 0,5 % bis etwa 2 % des Extraktgewichts umfasst.

17. Der Chicoréeextrakt für den Gebrauch nach Anspruch 15, wobei die erhöhte Glutathion-S-Transferase-Aktivität zu einem erhöhten Glutathionniveau führt.

## Revendications

1. Quantité biologiquement efficace d'un extrait de chicorée pour une utilisation dans la réduction du risque de cancer chez un mammifère, où
(i) l'extrait de chicorée est extrait à partir de chicorées par hydrolyse acide et extraction à l'aide d'un solvant avec de l'acétate d'éthyle,
(ii) l'extrait de chicorée comprend un agent phytochimique qui induit l'activité glutathion-S-transférase chez le mammifère.

2. Extrait de chicorée pour une utilisation selon la revendication 1, où l'extrait de chicorée est extrait à partir de la racine de chicorée, de la pulpe de chicorée et de combinaisons de celles-ci.

3. Extrait de chicorée pour une utilisation selon la revendication 1, où l'agent phytochimique est choisi dans le groupe comprenant un antioxydant, des catéchines et des combinaisons de ceux-ci.

4. Extrait de chicorée pour une utilisation selon la revendication 1, où l'activité glutathion-S-transférase est capable d'activer la détoxification dans un tissu du mammifère.

5. Extrait de chicorée pour une utilisation selon la revendication 1, où l'activité glutathion-S-transférase est capable de stimuler une défense antioxydante dans un tissu du mammifère.

6. Extrait de chicorée pour une utilisation selon la revendication 1, où l'extrait de chicorée est formulé dans une composition nutritionnelle choisie dans le groupe comprenant un complément nutritionnel, un produit alimentaire nutritionnellement complet, une préparation alimentaire, un produit de céréale et un aliment pour animaux de compagnie.

7. Extrait de chicorée pour une utilisation selon la revendication 6, où l'aliment pour animaux de compagnie comprend une matrice d'amidon.

8. Produit alimentaire pour animaux de compagnie pour une utilisation dans la réduction du risque de cancer chez un mammifère comprenant un extrait de chicorée extrait à partir de chicorées par hydrolyse acide et extraction à l'aide d'un solvant avec de l'acétate d'éthyle, où l'extrait de chicorée comprend un agent phytochimique qui induit l'activité glutathion-S-transférase chez le mammifère.

9. Produit alimentaire pour animaux de compagnie pour une utilisation selon la revendication 8, où le produit comprend une quantité d'au moins 0,5 % de l'extrait de chicorée sur une base de poids sec.

10. Produit alimentaire pour animaux de compagnie pour une utilisation selon la revendication 8, où l'agent phytochimique est choisi dans le groupe comprenant un antioxydant, des catéchines et des combinaisons de ceux-ci.

11. Procédé de préparation d'un produit alimentaire nutritionnel capable de réduire un risque de cancer chez un mammifère, le procédé comprenant les étapes consistant à :
traiter de la chicorée par hydrolyse acide et extraction à l'aide d'un solvant avec de l'acétate d'éthyle pour former un extrait de chicorée comprenant un agent phytochimique capable d'induire l'activité glutathion-S-transférase chez le mammifère ; et
traiter l'extrait et un ou plusieurs composants alimentaires pour former le produit alimentaire nutritionnel qui comprend au moins 0,5 % en poids de l'extrait.

12. Procédé selon la revendication 11, dans lequel l'extrait de chicorée est traité par dégraissage de chicorée pour former un premier extrait végétal et ensuite traitement du premier extrait végétal avec de l'acétate d'éthyle par l'intermédiaire d'une hydrolyse acide pour former l'extrait végétal.

13. Quantité biologiquement efficace d'un extrait de chicorée extrait à partir de chicorées par hydrolyse acide et extraction à l'aide d'un solvant avec de l'acétate d'éthyle qui contient un agent phytochimique pour une utilisation dans l'augmentation de la détoxification d'un tissu d'un mammifère, où l'agent phytochimique induit l'activité glutathion-S-transférase chez le mammifère.

14. Extrait de chicorée pour une utilisation selon la revendication 13, où la composition nutritionnelle comprend environ 0,5 % à environ 2 % en poids de l'extrait.

15. Quantité biologiquement efficace d'un extrait de chicorée qui contient un agent phytochimique pour une utilisation dans la stimulation d'une défense antioxydante dans un tissu d'un mammifère, où l'agent phytochimique induit une activité glutathion-S-transférase chez le mammifère, et où l'extrait de chicorée est extrait à partir de chicorées par hydrolyse acide et extraction à l'aide d'un solvant avec de l'acétate d'éthyle.

16. Extrait de chicorée pour une utilisation selon la revendication 15, où la composition nutritionnelle comprend environ 0,5 % à environ 2 % en poids de l'extrait.

17. Extrait de chicorée pour une utilisation selon la revendication 15, où l'augmentation de l'activité glutathion-S-transférase entraîne une augmentation du taux de glutathion.
